# EUROPÄISCHE PATENTSCHRIFT

(11) **EP 0 646 571 B1**
(45) Veröffentlichungstag und Bekanntmachung des Hinweises auf die Patenterteilung: **11.06.1997**
(21) Anmeldenummer: 94114890.0
(22) Anmeldetag: 21.09.1994
(51) Int. Cl.: C07C 209/60, C07D 207/40, C07D 207/26, C07D 223/10, C07D 233/58

(54) **Verfahren zur Herstellung von N-Vinylverbindungen**
Process for the preparation of N-vinyl compounds
Procédé pour la préparation de composés N-vinyliques

(30) Priorität: 30.09.1993 DE 4333237
(43) Veröffentlichungstag der Anmeldung: 05.04.1995
(73) Patentinhaber: BASF Aktiengesellschaft, 67063 Ludwigshafen (DE)
(72) Erfinder: Heider, Marc, Dr., D-67433 Neustadt (DE); Henkelmann, Jochem, Dr., D-68165 Mannheim (DE); Ruehl, Thomas, Dr., D-67227 Frankenthal (DE)

(56) Entgegenhaltungen:
- EP-A- 0 512 656
- WO-A-89/09210
- DE-C- 1 018 396

## Beschreibung

Die vorliegende Erfindung betrifft ein verbessertes Verfahren zur Herstellung von N-Vinylverbindungen der allgemeinen Formel I 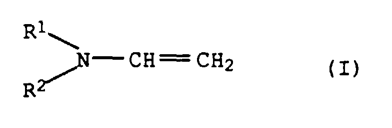 in der R¹ und R² unabhängig voneinander Wasserstoff, C₁- bis C₂₀-Alkyl, C₅- bis C₈-Cycloalkyl, C₇- bis C₁₈-Aralkyl, C₆- bis C₁₈-Aryl oder C₁- bis C₂₀-Acyl bedeuten, wobei einer der Reste R¹ oder R² von Wasserstoff verschieden sein muß, oder die Gruppierung R¹-N-R² Bestandteil eines gesättigten, ungesättigten oder aromatischen heterocyclischen Fünf- bis Siebenringes ist, der noch bis zu zwei weitere Heteroatome aus der Gruppe Stickstoff, Sauerstoff und Schwefel oder bis Ketofunktionen enthalten und zusätzlich zu zwei benzanelliert sein kann,
durch Umsetzung von NH-Verbindungen der allgemeinen Formel II 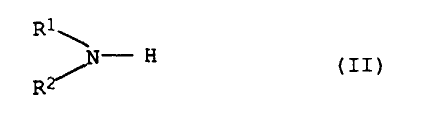 mit Acetylen bei Temperaturen von 50 bis 250°C und Drücken von 1 bis 30 bar in Gegenwart von Verbindungen der Platinmetalle als Katalysatoren.

N-Vinylverbindungen sind als Monomere für die Herstellung von Polymerisaten, die auf verschiedensten Gebieten Anwendung finden, von Interesse. Derartige Polymerisate werden beispielsweise als Mittel zur Klärung von Getränken, als Filmbildner in Haarfestigungsmitteln oder als Farbstoff-Übertragungsinhibitoren in Waschmittel eingesetzt.

Aus der EP-A 512 656 (1) ist ein Verfahren zur Herstellung von Vinyl-Derivaten von Brönsted-Säuren, d.h. wasserstoffaktiven Verbindungen, beispielsweise sekundären aromatischen Aminen, primären aliphatischen Aminen, Amiden oder Imiden, durch Umsetzung mit Acetylen in der Gasphase bei 40 bis 250°C unter Druck in Gegenwart von nullwertigem Ruthenium auf einem Trägermaterial als Katalysator bekannt, wobei das Acetylen mit Stickstoff im Vol.-Verhältnis von ca. 0,07 : 1 verdünnt und wobei der Verbrauch des Acetylens während der Umsetzung nicht kontrolliert wird. Auf diese Weise werden z.B. N-Vinylsuccinimid, N-Vinyl-2-pyrrolidon oder N-Vinyl-ε-caprolactam hergestellt.

In der Literaturstelle Organometallics Vol. 2 (1983), S. 1689-1691 (2) offenbaren M. Rotem und Y. Shvo die Verwendung von bestimmten Rutheniumcarbonylen, z.B. Ru₃(CO)₁₂, als Katalysator-Vorstufen bei der Addition von aliphatischen oder aromatischen Carbonsäuren an di- oder monosubstituierte Acetylene unter Ausbildung von Vinylestern. Dabei werden die Rutheniumcarbonyle ohne Trägermaterialien eingesetzt, die Umsetzung erfolgt in Toluol bei 145°C unter Druck. Nebenbei wird erwähnt, daß sich bei dieser Umsetzung Ruthenium(III)-chlorid als vollkommen inaktiv erwiesen habe.

Aufgabe der vorliegenden Erfindung war es, ein effektiveres und wirtschaftlicheres Verfahren mit einer besseren Raum-Zeit-Ausbeute bereit bereitzustellen, außerdem sollte der Umsatz des Acetylens besser kontrollierbar sein.

Demgemäß wurde das eingangs definierte Verfahren gefunden, welches dadurch gekennzeichnet ist, daß man das Acetylen im Reaktor mit einem inerten Gas im Vol.-Verhältnis von 6 : 1 bis 0,5 : 1 verdünnt und Acetylen in dem Maße, wie es verbraucht wird, nachgepreßt.

Dem Fachmann ist bekannt, daß das Arbeiten mit Acetylen unter Druck, insbesondere bei höheren Temperaturen, ein Sicherheitsrisiko beinhalten, da sich das Acetylen explosionsartig zersetzen kann. Daher ist man bisher bestrebt gewesen, in solchen Systemen das Acetylen mit einem inerten Medium, z.B. Stickstoff, stark zu verdünnen, wie auch in (1) beschrieben. Deshalb war es um so überraschender, daß bei der vorliegenden Erfindung das Acetylen nur mit geringen Mengen eines inerten Gases verdünnt zu werden braucht, um das Sicherheitsrisiko einer Explosion auszuschalten. Die Möglichkeit, den Acetylenverbrauch kontrollieren zu können, erhöht ebenfalls die Sicherheit des Verfahrens.

Als inerte Gase zur Verdünnung des Acetylens eignen sich vor allem Argon, Kohlenmonoxid, Propan und insbesondere Stickstoff.

Man verdünnt das Acetylen mit dem inerten Gas vorzugsweise im Vol.-Verhältnis von 5 : 1 bis 1 : 1, insbesondere von 4 : 1 bis 2 : 1. Als Maßzahlen für die Volumina der Gase werden hier meist ihre Partialdrücke im Reaktor verwendet.

Als Katalysatoren kommen Verbindungen von null- bis achtwertigen Platinmetallen, d.h. von Ruthenium, Rhodium, Palladium, Osmium, Iridium und Platin, in Betracht. Als Platinmetalle werden hierbei Palladium, Osmium und insbesondere Ruthenium bevorzugt. Besonders bevorzugt werden Palladium(II)-, Osmium(III)- und Ruthenium(III)-Verbindung, z.B. PdCl₂, OsCl₃, Ru(III)-acetylacetonat und insbesondere RuCl₃, Man kann aber auch Platinmetalle anderer Wertigkeitsstufen einsetzen, z.B. nullwertige Rutheniumcarbonyle wie Ru₃(CO)₁₂.

Die Katalysatoren können auf Trägermaterialien wie Aktivkohle, Kieselgel, Aluminiumoxid oder Ionenaustauscherharzen aufgebracht werden, bevorzugt wird jedoch ein trägermaterialfreier Einsatz.

Die Katalysatoren werden zweckmäßigerweise in den hierfür üblichen Mengen, also etwa von 0,05 bis 10 mol-%, vorzugsweise 0,2 bis 5 mol-%, bezogen auf ein Äquivalent NH in den Verbindungen II, eingesetzt.

Die Umsetzung wird zweckmäßigerweise in einem inerten organischen Lösungsmittel durchgeführt. Hierfür eignen sich vor allem höhersiedende aliphatische oder aromatische Kohlenwasserstoffe, z.B. Toluol oder Xylole, höhersiedende aliphatische oder aromatische Halogenkohlenwasserstoffe, z.B. Chlorbenzol oder Dichlorbenzole, oder Amide, z.B. N-Methylpyrrolidon oder Dimethylformamid. Man verwendet diese Lösungsmittel üblicherweise in einer Menge von 50 bis 800 Gew.-%, insbesondere 100 bis 500 Gew.-%, bezogen auf die Menge eingesetzter NH-Verbindung II.

Die Umsetzung der NH-Verbindungen II mit Acetylen (C₂H₂) erfolgt in der Regel in einem geschlossenen druckfesten Reaktor (Autoklav). Man arbeitet bei Temperaturen von 140 bis 180°C, und bei Drücken von 15 bis 22 bar. Die Umsetzung benötigt unter diesen Bedingungen etwa 3 bis 30 Stunden, meist 5 bis 25 Stunden.

Die Aufarbeitung des Reaktionsansatzes erfolgt im einfachsten Fall zweckmäßigerweise durch Destillation des rohen Vinylierungsproduktes. Der eingesetzte Katalysator kann wiederverwendet werden.

Als geradkettige oder verzweigte C₁- bis C₂₀-Alkylreste für R¹ und R² eignen sich beispielsweise Methyl, Ethyl, n-Propyl, iso-Propyl, n-Butyl, iso-Butyl, sec.-Butyl, tert.-Butyl, n-Amyl, iso-Amyl, sec.-Amyl, tert.-Amyl, Neopentyl, n-Hexyl, n-Heptyl, n-Octyl, 2-Ethylhexyl, n-Nonyl, iso-Nonyl, n-Decyl, n-Undecyl, n-Dodecyl, n-Tridecyl, iso-Tridecyl, n-Tetradecyl, n-Pentadecyl, n-Hexadecyl, n-Heptadecyl, n-Octadecyl und n-Eicosyl. Bevorzugt werden hiervon C₁- bis C₄-Alkylreste.

Als C₅- bis C₈-Cycloalkylreste für R¹ und R² kommen vor allem C₅-bis C₆-Cycloalkyl wie Cyclopentyl und Cyclohexyl, daneben der auch Cycloheptyl, Cyclooctyl, Methylcyclopentyl, Dimethylcyclopentyl, Methylcyclohexyl, Ethylcyclohexyl und Dimethylcyclohexyl in Betracht.

Als C₇- bis C₁₈-Aralkylreste für R¹ und R² eignen sich beispielsweise Naphthylmethyl, Diphenylmethyl oder Methylbenzyl, insbesondere jedoch C₇- bis C₁₈-Phenylalkyl wie 1-Phenylethyl, 2-Phenylethyl, 1-Phenylpropyl, 2-Phenylpropyl, 3-Phenylpropyl, 2-Phenylprop-2-yl, 4-Phenylbutyl, 2,2-Dimethyl-2-phenylethyl, 5-Phenylamyl, 10-Phenyldecyl, 12-Phenyldodecyl oder vor allem Benzyl.

Als C₆- bis C₁₈-Arylreste für R¹ und R² kommen vor allem ein- oder zweikernige Reste in Betracht, die noch ein bis drei C₁- bis C₄-Alkylgruppen als Substituenten tragen können. Insbesondere sind hier unsubstituiertes Phenyl, o-, m- und p-Tolyl, p-Ethylphenyl, Cumyl, p-tert.-Butylphenyl und α- und β-Naphthyl zu nennen.

Als C₁- bis C₂₀-Acylreste eignen sich vor allem solche, bei denen die Carbonylgruppe direkt benachbart zum N-Atom ist, so daß Amid-, Lactam- oder Imid-Strukturen vorliegen. Beispiel für C₁-bis C₂₀-Acylreste sind Formyl, Acetyl, Propionyl und Butyryl.

In einer bevorzugten Ausführungsform werden solche N-Vinylverbindungen I nach dem erfindungsgemäßen Verfahren hergestellt, bei denen die Gruppierung R¹-N-R² Bestandteil eines gesättigten, ungesättigten oder aromatischen heterocyclischen Fünf- bis Siebenringes ist, der noch ein weiteres Stickstoffatom oder ein Sauerstoffatom oder bis zu zwei Ketofunktionen enthalten und zusätzlich benzanelliert sein kann.

Als umzusetzende NH-Verbindungen II kommen beispielsweise in Betracht:
- offenkettige aliphatische primäre oder vorzugsweise sekundäre Amine wie Dimethylamin, Diethylamin oder Di-n-butylamin;
- cycloalkylsubstituierte Amine wie Cyclohexylamin;
- aryl- und aralkylsubstituierte Amine wie Anilin, N-Methylanilin, o-, m- oder p-Toluidin, α- oder β-Naphthylamin und Benzylamin;
- offenkettige Carbonsäureamide wie Formamid, N-Methylformamid, Acetamid oder Propionamid;
- Lactame wie 2-Pyrrolidon, δ-Butyrolactam oder ε-Caprolactam;
- offenkettige oder vorzugsweise cyclische Dicarbonsäureimide wie Succinimid, Maleinsäureimid oder Phthalsäureimid;
- gesättigte oder teilweise ungesättigte Stickstoffetherocyclen wie Pyrrolidin, 2- oder 3-Pyrrolin, Piperidin, Morpholin, Indolin oder Isoindolin;
- aromatische Stickstoffheterocyclen wie Imidazol, Pyrrol, Pyrazol, 1,2,3- oder 1,2,4-Triazol, Indol, Isoindol, Benzimidazol oder 1H-Azepin.

Es können auch NH-Verbindungen II mit mehreren NH-Gruppen eingesetzt werden, z.B. aliphatische Diamine wie N,N'-Dialkyl-α,ω-diaminoalkane oder gesättigte Heterocyclen wie Imidazolodin oder Pyrazan.

Besonders bevorzugt werden als umzusetzende NH-Verbindungen II 2-Pyrrolidon, Imidazol und ε-Caprolactam.

Man setzt üblicherweise pro NH-Gruppe in den Verbindungen II 0,95 bis 1,1 mol, vorzugsweise 1,0 bis 1,05 mol Acetylen ein, was durch die erfindungsgemäße Verfahrensführung leicht zu kontrollieren ist.

Das erfindungsgemäße Verfahren zur Vinylierung der NH-Verbindungen II kann auf eine Vielzahl verschiedener NH-Verbindungen ohne Probleme angewandt werden. Die N-Vinylverbindungen I können in sehr guten Ausbeuten und weitestgehend frei von Verunreinigungen erhalten werden. Sonst häufig auftretende Nebenreaktionen wie Verseifung von Carbonamid-Gruppierungen oder Polymerisation werden in der Regel beim erfindungsgemäßen Verfahren nicht beobachtet.

### Beispiele 1 bis 7

Eine Mischung aus a Gramm NH-Verbindung, b Gramm Lösungsmittel und c mmol Platinmetallverbindung als Katalysator wurden in einen 300 ml-Hochdruckautoklaven eingefüllt. Nach Spülen mit Stickstoff wurde der Autoklav erhitzt, es wurden 5 bar Stickstoff und Acetylen bis zu einem Gesamtdruck von 20 bar aufgepreßt (Vol.-Verhältnis C₂H₂ zu N₂ 3 : 1). In dem Maße, in dem Acetylen verbraucht wurde, wurde es nachgepreßt. Nachdem die Reaktion beendet war, erhielt man durch Destillation des Rohprodukts die entsprechende N-Vinylverbindung. Einzelheiten können der nachstehenden Tabelle entnommen werden.

| Bsp. Nr. | NH-Verbindung Menge a [g] | Katalysator Menge c [mmol] | | Temperatur [°C] | Lösungsmittel Menge b [g] | | Reaktionsdauer [h] | Ausbeute [%] |
|---|---|---|---|---|---|---|---|---|
| 1 | 2-Pyrrolidon 75 | RuCl₃ | 6,5 | 150 | Toluol | 75 | 24 | 87 |
| 2 | 2-Pyrrolidon 30 | Ru(acac)₃ | 5,3 | 160 | Toluol | 120 | 14 | 77 |
| 3 | 2-Pyrrolidon 100 | RuCl₃ | 9,6 | 150 | - | - | 20 | 82 |
| 4 | 2-Pyrrolidon 30 | OsCl₃ | 1,7 | 150 | Toluol | 120 | 18 | 44 |
| 5 | Imidazol 30 | RuCl₃ | 1,3 | 150 | Toluol | 120 | 24 | 64 |
| 6 | Succinimid 30 | RuCl₃ | 9,5 | 150 | NMP | 120 | 7 | 72 |
| 7 | 2-Pyrrolidon 75 | Ru(acac)₃ *) | 2,5 | 160 | Toluol | 75 | 24 | 85 |
| acac = Acetylacetonat, NMP = N-Methylpyrrolidon | | | | | | | | |

| | | | | | | | | |
|---|---|---|---|---|---|---|---|---|
| *) 5 mmol Pyridin zugesetzt | | | | | | | | |

## Patentansprüche

1. Verfahren zur Herstellung von N-Vinylverbindungen der allgemeinen Formel I 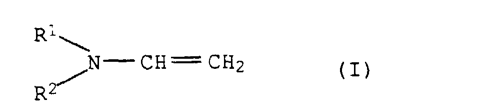 in der R¹ und R² unabhängig voneinander Wasserstoff, C₁- bis C₂₀-Alkyl, C₅- bis C₈-Cycloalkyl, C₇- bis C₁₈-Aralkyl, C₆- bis C₁₈-Aryl oder C₁- bis C₂₀-Acyl bedeuten, wobei einer der Reste R¹ oder R² von Wasserstoff verschieden sein muß, oder die Gruppierung R¹-N-R² Bestandteil eines gesättigten, ungesättigten oder aromatischen heterocyclischen Fünf- bis Siebenringes ist, der noch bis zu zwei weitere Heteroatome aus der Gruppe Stickstoff, Sauerstoff und Schwefel oder bis zu zwei Ketofunktionen enthalten und zusätzlich benzanelliert sein kann,
durch Umsetzung von NH-Verbindungen der allgemeinen Formel II 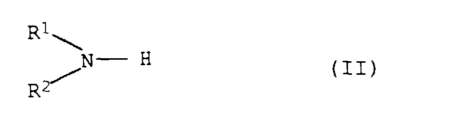 mit Acetylen bei Temperaturen von 140 bis 180°C und Drücken von 15 bis 22 bar in Gegenwart von Verbindungen der Platinmetalle als Katalysatoren, dadurch gekennzeichnet, daß man das Acetylen im Reaktor mit einem inerten Gas im Vol.-Verhältnis von 6 : 1 bis 0,5 : 1 verdünnt und Acetylen in dem Maße, wie es verbraucht wird, nachpreßt.

2. Verfahren zur Herstellung von N-Vinylverbindungen I nach Anspruch 1, dadurch gekennzeichnet, daß man das Acetylen im Reaktor mit Stickstoff im Vol.-Verhältnis von 5 : 1 bis 1 : 1 verdünnt.

3. Verfahren zur Herstellung von N-Vinylverbindungen I nach Anspruch 1 oder 2 unter Verwendung von Palladium(II)-, Osmium(III)- oder Ruthenium(III)-Verbindungen als Katalysatoren.

4. Verfahren zur Herstellung von N-Vinylverbindungen I nach den Ansprüchen 1 bis 3, wobei man die Katalysatoren trägermaterialfrei einsetzt.

5. Verfahren zur Herstellung von N-Vinylverbindungen I nach den Ansprüchen 1 bis 4, wobei man die Umsetzung in einem inerten organischen Lösungsmittel durchführt.

6. Verfahren zur Herstellung von N-Vinylverbindungen I nach den Ansprüchen 1 bis 5, bei denen die Gruppierung R¹-N-R² Bestandteil eines gesättigten, ungesättigten oder aromatischen heterocyclischen Fünf- bis Siebenringes ist, der noch ein weiteres Stickstoffatom oder ein Sauerstoffatom oder bis zu zwei Ketofunktionen erhalten und zusätzlich benzanelliert sein kann.

7. Verfahren zur Herstellung von N-Vinylverbindungen I nach Anspruch 6 durch Umsetzung von 2-Pyrrolidon, Imidazol oder ε-Caprolactam.

## Claims

1. A process for preparing N-vinyl compounds of the general formula I 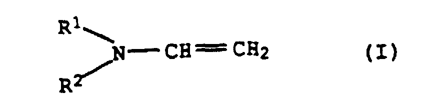 where R¹ and R² independently of one another are hydrogen, C₁-C₂₋-alkyl, C₅-C₈-cycloalkyl, C₇-C₁₈-aralkyl, C₆-C₁₈-aryl or C₁-C₂₀-acyl, and one of the radicals R¹ and R² must be other than hydrogen, or the group R¹-N-R² is part of a saturated, unsaturated or aromatic heterocyclic five- to seven-membered ring which can also include up to two further heteroatoms from the group consisting of nitrogen, oxygen and sulfur or up to two keto functions and can, in addition, be benzo-fused,
by reacting NH compounds of the general formula II 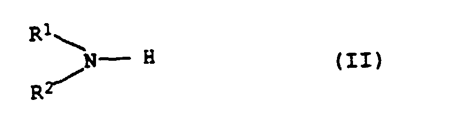 with acetylene at temperatures from 140 to 180°C and at pressures of from 15 to 22 bar in the presence of compounds of the platinum metals as catalysts, which comprises diluting the acetylene in the reactor in an inert gas in a volume ratio of from 6:1 to 0.5:1 and injecting further acetylene at the rate at which it is consumed.

2. A process for preparing N-vinyl compounds I as claimed in claim 1, wherein the acetylene in the reactor is diluted with nitrogen in a volume ratio of from 5:1 to 1:1.

3. A process for preparing N-vinyl compounds I as claimed in claim 1 or 2 using palladium(II) compounds, osmium(III) compounds or ruthenium(III) compounds as catalysts.

4. A process for preparing N-vinyl compounds I as claimed in any of claims 1 to 3, where the catalysts are employed without support material.

5. A process for preparing N-vinyl compounds I as claimed in any of claims 1 to 4, where the reaction is carried out in an inert organic solvent.

6. A process for preparing N-vinyl compounds I as claimed in any of claims 1 to 5, where the group R¹-N-R² is part of a saturated, unsaturated or aromatic heterocyclic five- to seven-membered ring which can also contain a further nitrogen atom or an oxygen atom or up to two keto functions and can, in addition, be benzofused.

7. A process for preparing N-vinyl compounds I as claimed in claim 6 by reacting 2-pyrrolidone, imidazole or ε-caprolactam.

## Revendications

1. Procédé de préparation de composés N-vinyliques de formule générale I 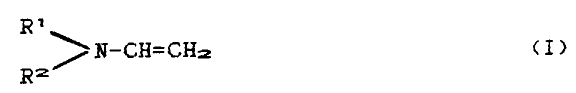 dans laquelle R¹ et R² représentent chacun, indépendamment l'un de l'autre, un atome d'hydrogène ou un reste alkyle en C₁-C₂₀, cycloalkyle en C₅-C₈, aralkyle en C₇-C₁₈, aryle en C₆-C₁₈- ou acyle en C₁-C₂₀, l'un des restes R¹ et R² devant être différent d'un atome d'hydrogène, ou le groupement R¹-N-R² fait partie d'un noyau hétérocyclique à 5-7 chaînons saturé, insaturé ou aromatique, qui peut encore contenir jusqu'à deux autres hétéroatomes du groupe de l'azote, de l'oxygène et du soufre ou jusqu'à deux fonctions céto et qui peut être en plus benzocondensé,
par réaction de composés NH de formule générale II 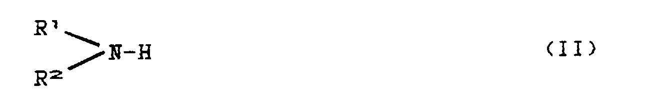 avec de l'acétylène à des températures de 140 à 180°C et sous des pressions de 15 à 22 bar, en présence de composés des métaux du groupe du platine en tant que catalyseurs, caractérisé en ce que l'on dilue l'acétylène dans le réacteur avec un gaz inerte dans un rapport volumique de 6:1 à 0,5:1 et on recomprime de l'acétylène dans la mesure où il est consommé.

2. Procédé de préparation de composés N-vinyliques I selon la revendication 1, caractérisé en ce que l'on dilue l'acétylène dans le réacteur avec de l'azote dans un rapport volumique de 5:1 à 1:1.

3. Procédé de préparation de composés N-vinyliques I selon la revendication 1 ou 2 avec utilisation de composés du palladium(II), de l'osmium(III) ou du ruthénium(III) en tant que catalyseurs.

4. Procédé de préparation de composés N-vinyliques I selon l'une quelconque des revendications 1 à 3, dans lequel on utilise les catalyseurs sans matière de support.

5. Procédé de préparation de composés N-vinyliques I selon l'une quelconque des revendications 1 à 4, dans lequel on conduit la réaction dans un solvant organique inerte.

6. Procédé de préparation de composés N-vinyliques I selon l'une quelconque des revendications 1 à 5, dans lequel le groupement R¹-N-R² fait partie d'un noyau hétérocyclique à 5-7 chaînons saturé, insaturé ou aromatique, qui peut encore contenir un autre atome d'azote, un atome d'oxygène ou jusqu'à deux fonctions céto et qui peut être en plus benzocondensé.

7. Procédé de préparation de composés N-vinyliques I selon la revendication 6 par réaction de 2-pyrrolidone, d'imidazole ou d'ε-caprolactame.
